# EUROPEAN PATENT APPLICATION

(11) **EP 4 840 395 A1**
(43) Date of publication of application: **18.11.2026**
(21) Application number: 25176561.6
(22) Date of filing: 15.05.2025
(51) Int. Cl.: A61B 5/08, A61B 5/00, A61B 10/00

(54) **ORAL CARE DEVICE**

(71) Applicant: Koninklijke Philips N.V., 5656 AE Eindhoven (NL)
(72) Inventor: VAN ACHT, Victor Martinus Gerardus, Eindhoven (NL); VAN DEN DUNGEN, Wilhelmus Andreas Marinus Arnoldus Maria, Eindhoven (NL); KOOIJMAN, Gerben, Eindhoven (NL); VAN LIESHOUT, Ron Martinus Laurentius, Eindhoven (NL)
(74) Representative: Philips Intellectual Property & Standards

(57) **Abstract**

Proposed concepts thus aim to provide schemes, solutions, concepts, designs, methods and systems pertaining to an oral care device. In particular, embodiments aim to provide an oral care device, which, by including a concentrator component distinct and separate from a sensor (though still releasably attachable/detachable to a sensor), is able to efficiently capture selected molecules from the mouth of a user for subsequent analysis by a separate sensor at a later time. In this way, gas/liquid samples can be efficiently collected from a user's mouth, e.g., during brushing, for analysis without requiring the actual sensor to miniaturized within the oral care device to, for example, analyse the gas/liquid while the oral care device is inside a user's oral cavity.

## Description

### FIELD OF THE INVENTION

This invention relates to the field of oral care devices.

### BACKGROUND OF THE INVENTION

Oral-systemic health monitoring identifies connections between oral health and overall health. Using an oral care device for measuring gases in the mouth enables non-invasive monitoring that is frequent (typically twice a day) and consistent over time, facilitating the detection of early signs of systemic diseases. Detecting specific gas compounds helps monitor conditions such as diabetes, respiratory diseases, and infections. For gases to be analyzed inside a user's oral cavity, however, extreme miniaturization of a gas sensor would be required.

For the detection of gas molecules, a (pre-)concentrator can be used. A pre-concentrator is a chemical compound that binds molecules of interest (e.g., volatile organic compounds, VOCs) in one operating regime and releases the previously collected molecules in another operating regime. For example, the concentrator may be in one operating regime at a low temperature (binding molecules) and another operating regime at a high temperature (releasing the bound molecules). Typically, the time that the device operates in the binding regime is much longer than the time that the device operates in the releasing regime, and thus it works as a concentrator. Typically, a concentrator is used to increase the sensitivity of the overall gas concentration measurement system. Additionally, different gas molecules can have different release regimes (e.g., they are released at different temperatures) and thus this can be used to increase the specificity of the overall gas sensing system.

### SUMMARY OF THE INVENTION

The invention is defined by the claims.

According to examples in accordance with an aspect of the invention, there is provided an oral care device.

The oral care device comprises: a concentrator component configured, whilst in or near a mouth of a user, to releasably bond with selected molecules from the mouth of the user; wherein the concentrator component is configured to be releasably attachable to a sensor configured to analyze selected molecules bonded with the concentrator component.

Proposed concepts thus aim to provide schemes, solutions, concepts, designs, methods and systems pertaining to an oral care device. In particular, embodiments aim to provide an oral care device which, by including a concentrator component distinct and separate from a sensor (though still releasably attachable/detachable to a sensor), is able to efficiently capture selected molecules from the mouth of a user for subsequent analysis by a separate sensor at a later time. In this way, gas/liquid samples can be efficiently collected from a user's mouth, e.g., during brushing, for analysis without requiring the actual sensor to be miniaturized within the oral care device to, for example, analyse the gas/liquid while the oral care device is inside a user's oral cavity.

In other words, typically a concentrator is part of a sensor, however, the inventors have realised that by having the concentrator be a distinct component which is only releasably attachable to a sensor rather than permanently part of one, the concentrator can be provided in a spatially-efficient way inside an oral care device, collecting gas/liquid to be analysed at a later time, without requiring any miniaturization or adjustment of the sensor itself.

Furthermore, another advantage of this oral care device is that without a concentrator component, a gas sensor would require a response time considerably shorter than two minutes (the average time of an oral brushing session) whereas by providing a standalone concentrator, the response time of a gas sensor essentially is allowed to be much longer than two minutes.

The invention thus facilitates a non-invasive and time-efficient way to monitor a user's health and thus maintain and/or improve it.

In some embodiments, the concentrator component is configured, in a first state, to bond with the selected molecules, and, in a second state, to release bonded molecules, and wherein the concentrator component is configured to be in the first state whilst in or near a mouth of a user. This provides an effective way to both collect selected molecules and release them such that they can be analysed by a sensor.

In some embodiments, the concentrator component is configured to be in the second state responsive to: exposure of the concentrator component to a predetermined surrounding temperature; exposure of the concentrator component to a light of a predetermined intensity and/or wavelength; exposure of the concentrator component to a predetermined surrounding air pressure; exposure of the concentrator component to a predetermined surrounding partial pressure of the selected molecules; exposure of the concentrator component to a solvent; exposure of the concentrator component to a mechanical stress; and/or exposure of the concentrator component to an electric field of a predetermined strength. These all provide suitable mechanisms for inducing the release of bonded molecules.

In some embodiments, the concentrator component comprises a thin film configured to bond with the selected molecules. This has been found to be a particularly effective form of concentrator component for the present purpose.

In some embodiments, the thin film comprises at least one of: a metal organic framework, MOF, material; a polymer; and/or biological anti-bodies. These have been found to be particularly materials for use in thin films for the present purpose.

In some embodiments, the device further comprises a semi-permeable membrane covering the concentrator component and configured to permit passage of gas and prevent the passage of liquid. This thus protects the concentrator component against saliva whilst allowing passage of gas.

In some embodiments, the device further comprises a temporary layer covering a part of the concentrator component and configured to dissolve after a period of use of the oral care device thereby exposing the part of the concentrator component. This facilitates tracking of the selected molecules over time.

In some embodiments, the oral care device further comprises: a body; a head mounted on the body of the oral care device; and a plurality of cleaning elements mounted on the head; wherein the concentrator component forms part of at least one of the plurality of cleaning elements, and/or wherein the concentrator component is provided on a surface of the brush head. The concentrator forming part of the cleaning elements (e.g., bristles) may be useful, for instance, when bonding is activated through certain mechanical deformation, and the concentrator being part of the surface of the brush head, for instance, the back, may help reduce contact with oral surfaces, toothpaste and saliva.

According to another aspect of the invention, there is provided a system comprising the oral care device of any herein-described embodiment; and a sensor configured to: receive the concentrator component; release the molecules bonded with the concentrator component; and analyse the extracted molecules.

In some embodiments, the sensor and/or the concentrator component comprises an attaching mechanism configured to releasably attach and detach the sensor and concentrator component to and from one another. This provides an effective way to facilitate the concentrator component being releasably attachable to the sensor.

In some embodiments, the sensor is integrated with a handle of the oral care device, or the sensor is integrated with a charging device for charging the oral care device, or the sensor is integrated with a sanitizer device for cleaning the oral care device. These are all suitable positions for the sensor allowing for unintrusive and time-efficient analysis of the bonded molecules.

In some embodiments, the sensor is further configured, responsive to releasing molecules bonded with the concentrator component, to apply a new concentrator component to the oral care device. This allows the concentrator component to essentially be replaced with a fresh concentrator component for further sampling the selected molecules in a user's mouth.

In some embodiments, the sensor is further configured, responsive to releasing molecules bonded with the concentrator component, to remove the concentrator component or deactivate the concentrator component. This further facilitates the replacement of the old concentrator component with a new one.

In some embodiments, the oral care device comprises a protective layer covering the concentrator component and configured to be impermeable to the selected molecules; and wherein the sensor is further configured to remove, responsive to releasing molecules bonded with the concentrator component, a part of the protective layer thereby exposing a corresponding part of the concentrator component. Removing parts of a protective layer to expose a part of the concentrator which has not yet been used essentially facilitates an alternative way to provide a fresh/new concentrator component.

According to another aspect of the invention, there is provided a system comprising the oral care device of any herein-described embodiment; and a preservation unit configured to: receive the concentrator component; apply a protective layer covering the concentrator component configured to be impermeable to the selected molecules; and apply a new concentrator component over the protective layer. In this system, a protective layer and new concentrator component are provided on a concentrator component which has already been used thereby allowing the layers of concentrator components to be peeled back over time to allow a time-varying analysis of selected molecules.

According to another aspect of the invention, there is provided a method for extracting selected molecules from a mouth of a user, comprising: providing, as part of an oral care device, a concentrator component configured, whilst in or near a mouth of a user, to releasably bond with selected molecules from the mouth of the user, wherein the concentrator component is configured to be releasably attachable to a sensor configured to analyse selected molecules bonded with the concentrator component; inserting the oral care device into the mouth of the user; and releasing the molecules bonded with the concentrator component.

Thus, there may be proposed concepts for an oral care device, and in particular, to an oral care device comprising a standalone concentrator component which is releasably attachable to a sensor for subsequent analysis of its releasably bonded selected molecules.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified block diagram of an oral care device according to a proposed embodiment;
Fig. 2 is a block diagram of an oral care device according to a proposed embodiment;
Fig. 3 is a simplified illustration of an oral care device according to a proposed embodiment;
Fig. 4 is a block diagram of system including a sensor according to a proposed embodiment;
Fig. 5 is a block diagram of a system including a sensor according to a proposed embodiment;
Fig. 6 is a block diagram of a system including a preservation unit according to a proposed embodiment; and
Fig. 7 is a simplified flow diagram of a method for extracting selected molecules from a mouth of a user according to a proposed embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to an oral care device. According to proposed concepts, a number of possible solutions may be implemented separately or jointly. That is, although these possible solutions may be described below separately, two or more of these possible solutions may be implemented in one combination or another.

Embodiments of the invention aim to provide an oral care device which, by including a concentrator component distinct and separate from a sensor (though still releasably attachable/detachable to a sensor), is able to efficiently capture selected molecules from the mouth of a user for subsequent analysis by a separate sensor at a later time. In this way, gas/liquid samples can be efficiently collected from a user's mouth, e.g., during brushing, for analysis without requiring the actual sensor to be miniaturized within the oral care device to, for example, analyse the gas/liquid while the oral care device is inside a user's oral cavity.

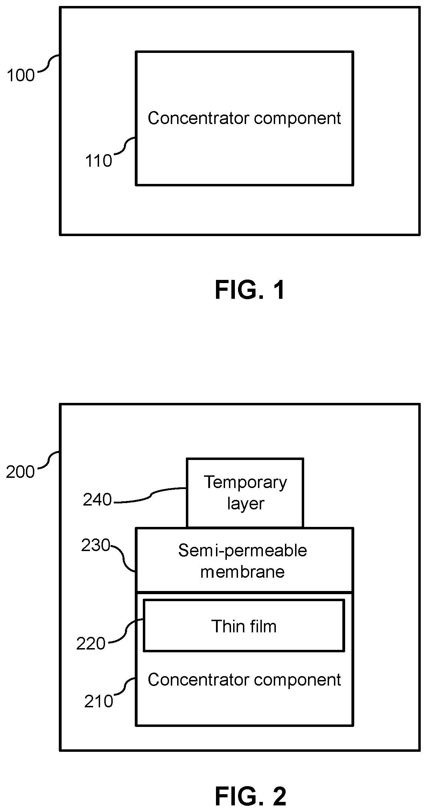

Referring now to Fig. 1, there is depicted a simplified block diagram of an oral care device 100 according to a proposed embodiment.

The oral care device 100 comprises a concentrator component 110. The concentrator component 110 (otherwise referred to simply as a concentrator or pre-concentrator) is configured, whilst in or near (i.e., within a predetermined distance of, e.g., within 5, 10, 15, or 20 cm) a mouth of a user, to releasably bond with selected molecules from the mouth of the user. This means that the concentrator will only (releasably) bond with target molecules, i.e., it selectively bonds with only molecules of a target group (the target group comprising one or more target compounds). To releasably bond is to be understood as only temporarily bonding such that the selected molecules can be released at a subsequent point in time, e.g., due to exposure to a particular light/gas/temperature, etc.

The concentrator component 110 is configured to be releasably attachable (i.e., controllably attachable and detachable by a user and/or system/device) to a sensor configured to analyse (the, if any) selected molecules bonded with the concentrator component. In other words, typically, the concentrator component is an integral part of a gas/liquid sensor for sensing selected/target molecules, however, in this invention, the concentrator component is standalone and forms part of the oral care device distinct from the sensor but is configured to still be releasably attachable to a sensor (e.g., via suitable attachment means) such that the selected molecules bonded to the standalone concentrator component can still be analysed at a later time and then, for example, re-used or replaced.

In this embodiment, the concentrator component 110 is configured, in a first ('bonding') state, to bond with the selected molecules, and, in a second ('releasing') state, to release any bonded molecules. The concentrator component 110 is configured to be in the first state whilst in or near a mouth of a user such that it can bond with selected molecules originating from the user's oral cavity. Furthermore, in this embodiment, the concentrator component 100 is configured to be in the second 'releasing' state responsive to: exposure of the concentrator component to a predetermined surrounding temperature (e.g., the state of the concentrator component is dependent on whether the surrounding temperature is above or below a predetermined threshold); exposure of the concentrator component to a light of a predetermined intensity and/or wavelength (e.g., the state of the concentrator component is dependent on whether it is exposed to light of an intensity above/below a predetermined threshold and/or of a wavelength/frequency above/below a predetermined threshold, for example UV light may be used to trigger release of the bonded molecules); exposure of the concentrator component to a predetermined surrounding air pressure (e.g., the state of the concentrator component is dependent on whether the surrounding air pressure is above or below a predetermined threshold, for example, the selected molecules may be released if exposed to a vacuum); exposure of the concentrator component to a predetermined surrounding partial pressure of the selected molecules (for example, capture at high partial pressure and release at low partial pressure); exposure of the concentrator component to a solvent (e.g., a specific solvent for the selected molecules); exposure of the concentrator component to a mechanical stress (e.g., a mechanical force resulting in deformation of a (nano)porous material, e.g., a vibration); and/or exposure of the concentrator component to an electric field of a predetermined strength (e.g., above a predetermined strength to promote absorbance or de-sorbance of molecules). These all provide suitable mechanisms for inducing the release of bonded molecules, though as the skilled person would appreciate, in other embodiments, any suitable mechanism can be used to induce a change in the concentrator component's state. The above mechanisms for changing the state of the concentrator component 110 can also be combined, for instance, using both vibration and heat may be particularly useful for releasing gases and microdroplets from the concentrator component.

Thus, ideally, while the concentrator component is in its first bonding state, it will be physically separated (i.e., physically remote / spatially isolated) from any sensor configured to analyse (the, if any) selected molecules bonded with the concentrator component; and, while the concentrator component is in its second releasing state, it will be attached to a sensor configured to analyse (the, if any) selected molecules bonded with the concentrator component.

It is noted that the oral care device 100 can comprise any suitable oral care device 100, for example, a toothbrush (manual or powered), mouthguard or mouth shield, U-shape toothbrush, i.e., a mouthpiece, etc. Essentially, the oral care device 100 can comprise any device which is intended to be placed in a user's oral cavity.

Regarding the selected molecules, example target molecules of interest (of clinical relevance) are typically volatile organic compounds (VOC's) such as: Acetone: elevated levels of acetone can indicate ketosis, which might be due to diabetes (particularly type 1 diabetes), dieting, or fasting; Isoprene: this compound is often linked to lipid metabolism, and variations in isoprene levels can be associated with oxidative stress or changes in metabolic activity, such as during exercise; Nitric Oxide (NO): increased levels can indicate airway inflammation, and it's commonly monitored in conditions like asthma; Methane: elevated levels can be associated with gastrointestinal issues, such as bacterial overgrowth in the intestines or certain types of infections; Ammonia: increased ammonia levels can be indicative of kidney dysfunction or liver disease and it is also produced during the breakdown of proteins by bacteria in the mouth such that high levels can be found in individuals with poor oral hygiene or oral infections; Carbon Monoxide (CO): elevated levels can result from smoking, environmental exposure, or hemolytic disorders; Hydrogen Cyanide (HCN): may be detected in smokers and can indicate exposure to cyanogenic compounds; Formaldehyde: can be related to oxidative stress and potential exposure to environmental pollutants; Hexanal and Pentanal: these compounds may be associated with lipid peroxidation, often reflecting oxidative stress or inflammatory processes; Ethane and Pentane: elevated levels can signal increased oxidative stress and lipid peroxidation, which is relevant in conditions like inflammatory diseases and cancer; Dimethyl Sulfide: raised levels can be indicative of liver disease and certain metabolic conditions and also contributes to halitosis and is produced by bacterial activity in the mouth such that elevated levels can be a sign of oral infections or poor oral hygiene; Hydrogen Sulfide (H₂S): produced by bacteria in the mouth, especially within dental plaque and periodontal pockets, and therefore elevated levels can contribute to halitosis and are often associated with periodontal disease and poor oral hygiene; Methyl Mercaptan: a sulfur-containing compound that is associated with bad breath and high levels are commonly found in individuals with gingivitis and periodontitis; Acetaldehyde: a byproduct of alcohol metabolism and bacterial activity in the mouth, elevated levels of which can indicate excessive alcohol consumption and increased risk of oral cancer; Ethanol: although normally transient, elevated levels can reflect recent alcohol consumption and chronic high levels can be related to systemic conditions such as liver disease and have implications for oral health; Methanol: produced in small quantities by bacterial activity in the mouth, elevated levels of which may indicate systemic metabolic abnormalities or external exposure; N-Butyric Acid: produced in the mouth by bacterial fermentation of food particles, and elevated levels can contribute to bad breath and are associated with poor oral hygiene; Propionic Acid: another fermentation product by oral bacteria, and thus elevated concentrations can contribute to halitosis and may indicate an imbalance in oral microbiota.

It should be noted that the concentrator component 110 may be reusable or may be intended for one-time use. In other words, depending on the configuration of the concentrator (e.g., chemical compounds used in the concentrator), it may be reused to capture a next batch of selected molecules after a previous batch has been released. Alternatively, the concentrator may be used only once, for example, if its ability to capture molecules is severely deteriorated after the first capture and release cycle. It should also be noted that the concentrator component 110 may be formed as a single element or may be formed from a plurality of distinct elements, physically disconnected from one another.

In short, conventionally, a (pre-)concentrator is used in a device for gas analysis as a submodule of a gas sensor (i.e., a gas analysis device), the gas sensor also including an analyser submodule. In an example of the invention, however, a molecular layer is applied to a dental care device that bonds specific gas molecules that are relevant for monitoring systemic health. In a subsequent step, the captured molecules are released again in a separate sensor or read-out unit for analysis. In this way, the molecular layer works as a pre-concentrator for gas analysis, where the pre-concentrator is a separate standalone device/component from the actual analyser.

Referring now to Fig. 2, there is depicted a block diagram of an oral care device 200 according to a proposed embodiment.

Compared to the oral care device 100 of Fig. 1, in this embodiment, the concentrator component 210 comprises a thin film configured to bond with the selected molecules. In this embodiment, the thin film specifically comprises a metal organic framework, MOF, material. A MOF is a type of material made from metal ions combined with organic molecules and creates a network on the molecular level that forms a porous structure. For example, beneficially, the MOF material could be applied on the fiber of bristle hairs (if the oral care device is a toothbrush) as most MOFs have the property that their pore size depends on the mechanical stress of the layer, e.g., that the pore size is increased when the MOF layer is stretched and decreased when the layer is squeezed. Thus, by repeatedly stretching and squeezing the MOF layer (e.g., during brushing), gas molecules can be trapped in the pores. In other embodiments, the thin film can alternative/additionally comprise at least one of: a polymer; and/or biological anti-bodies.

A thin film has been found to be particularly effective form of concentrator component for the present purpose though in other embodiments, the concentrator component 210 can take any suitable form for bonding with selected molecules. For example, as an alternative to using a thin film, anti-bodies can be used which are able to be made very specific to capture a particular molecule only. As another alternative, a metal-oxide (MOX) can be used, for example: Tin Oxide: this is particularly useful for detecting gases such as carbon monoxide, methane, hydrogen, and ethanol; Zinc Oxide: this is sensitive to gases such as hydrogen and ethanol; Titanium Dioxide: this is sensitive to gases such as nitrogen dioxide, hydrogen, and ammonia: Iron Oxide: this is sensitive to hydrogen sulphide and various hydrocarbons; Copper Oxide: this is sensitive to hydrogen and carbon monoxide; Nickel Oxide: this is sensitive to nitrogen dioxide and ammonia; and Indium Oxide: this is sensitive to ozone, nitrogen dioxide and carbon monoxide.

The oral care device 200 further comprises a semi-permeable membrane 230 covering (at least an outer surface (i.e., the surface which, in use, will be exposed to a user's mouth) of) the concentrator component 210 and configured to permit passage of gas and prevent the passage of liquid. This thus protects the concentrator component against saliva whilst allowing the passage of gas. In this embodiment, the target molecules can thus only comprise gas molecules.

The oral care device 200 further comprises a temporary layer 240 covering (at least) a part of the concentrator component (through the semi-permeable membrane 230) and configured to dissolve after a period of use of the oral care device thereby exposing the part of the concentrator component 210. This facilitates tracking of the selected molecules over time. The temporary layer 240 may be configured to dissolve due to saliva, abrasion (e.g., due to contact with oral surfaces) or simply due to the passage of time. It is noted that in this embodiment, which also includes a semi-permeable membrane 230, the temporary layer 240 is provided on top of the semi-permeable membrane, however, in other embodiments, the temporary layer 240 may also covered by the semi-permeable membrane 230, i.e., the temporary layer 240 may be provided inside or beneath the semi-permeable membrane 230. Of course, in embodiments which do not include a semi-permeable membrane 230, the temporary layer 240 can be provided directly on the concentrator component 210.

In a specific example of the invention, the concentrator component 210 forms part of bristles of a toothbrush (and can contain multiple parts, connected or disconnected to one another), and the temporary layer 240 comprises a coating on the bristles. The coating thickness per bristle can change (and/or the length of the bristles) such that the equilibrium of the part of the concentrator component that has been exposed earlier on (e.g., due to a thinner coating) will contain the largest time window and the equilibrium of the part of the concentrator component which is exposed last will have the smallest time-window, and thus by comparing these parts, how the equilibrium changes over time can be measured.

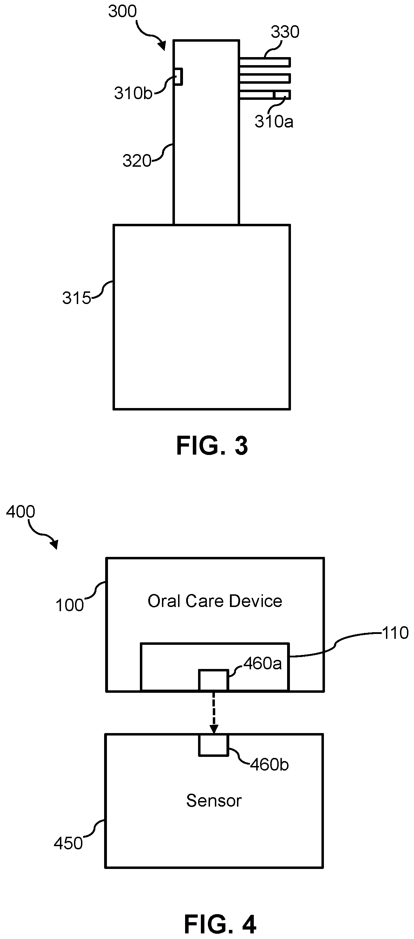

Referring now to Fig. 3, there is depicted a simplified illustration of an oral care device 300 according to a proposed embodiment. The oral care device 300 comprises a body 315, a head 320 (fixedly or releasably) mounted on the body of the oral care device, and a plurality of cleaning elements 330 mounted on the head 320. For instance, in this embodiment, the oral care device 300 is an automatic or manual toothbrush.

In this embodiment, a concentrator component, 310a and 310b, is present in both of the following positions (though in other embodiments, may be present in only one of the positions): forming part of at least one of the plurality of cleaning elements 330; and being provided on a surface of the brush head. Alternatively, in some embodiments, the concentrator component may be releasably attachable to the body 315 of the oral care device 300.

In other words, there are two preferred locations for the concentrator on a toothbrush head - either on the bristles themselves or on the ferrule. For example, the back of the ferrule would be maximally exposed to gasses in an oral cavity whilst having the least interaction with toothpaste and teeth. On the back of the ferrule, the concentrator component may also be in a beneficial position for easy application and/or for attaching to a sensor. The concentrator component being placed between bristles is also a preferred position. It should be noted that for some concentrator components, the capture and release mechanism for gas molecules is mechanical tension, and in these cases, it can be advantageous to apply the concentrator component on the bristles themselves so that during brushing, with the bristles moving and deforming, the bonding of gas molecules to the concentrator is triggered.

Referring now to Fig. 4, there is depicted a system 400 including a sensor 450 according to a proposed embodiment. The system 400 comprises an oral care device 100 according to any herein-described embodiment (in this embodiment, specifically the oral care device 100 of Fig. 1.), which itself comprises a concentrator component 110.

The sensor 450 is configured to: receive the concentrator component (i.e., releasably attach to the concentrator component); release (at least the selected) molecules bonded with the concentrator component; and analyse the extracted molecules (e.g., to determine the quantity of the selected molecules).

The skilled person would be aware of the many forms the sensor 450 could take for carrying out the above-described steps. For example, sensors capable of releasing molecules from a concentrator and analysing them may use thermal desorption to heat and liberate the trapped compounds, which are then directed to analytical components. Mass spectrometers or gas chromatographs can identify and quantify these molecules based on their mass-to-charge ratios or retention times, while photoionization detectors ionize volatile compounds using UV light for rapid detection. Other sensors, such as chemiresistors or field-effect transistors (FETs), detect specific chemical interactions by measuring changes in electrical properties, and optical techniques like infrared or Raman spectroscopy analyse molecular structures based on characteristic vibrational signatures. In short, the sensor 450 may be any gas and/or liquid sensor capable of releasing bonded molecules from a concentrator and analysing them.

In this embodiment, both the sensor 450 and the concentrator component 110 comprise an attaching mechanism, 460a and 460b, configured to releasably attach and detach the sensor and the concentrator component to and from one another. The skilled person would appreciate the myriad ways in which an attaching mechanism could be implemented. In other embodiments, only one of the sensor and the concentrator component may comprise an attaching mechanism, or neither (i.e., the attaching/detaching is facilitated in another way, e.g., glue).

In various embodiments, the sensor 450 can be integrated into one of the following components: a handle of the oral care device 100 (e.g., the handle of a toothbrush); a charging device for charging the oral care device 100 (e.g., a charging stand); or a sanitizer device for cleaning the oral care device 100 (e.g., a UV-light case for the oral care device). Alternatively, the sensor 450 can be a standalone device, not integrated into any other device/component, e.g., in a lab for a user to send the concentrator component to.

In this embodiment, the sensor 450 is configured to, responsive to releasing molecules bonded with the concentrator component 110, to remove the concentrator component or deactivate (i.e., make chemically inactive) the concentrator component. The sensor 450 is further configured to subsequently apply a new concentrator component to the oral care device 100, e.g., in the old concentrator component's place. This allows the concentrator component to essentially be replaced with a fresh concentrator component for further (more accurate and/or sensitive) sampling of the selected molecules in a user's mouth. It should be noted that, in other embodiments, these steps (i.e., removing/deactivating an old concentrator component an applying a new one) may not be performed by the sensor but rather by a further device / component, though this further device / component may be controlled based on the sensor's output.

It should be noted that a new concentrator component can still be applied even if the old concentrator component is not removed or deactivated, e.g., it can simply be applied over the top of the old concentrator component. This may allow analysis of the selected molecules over time, for instance, by analysing the different layers of concentrator components. It should also be noted that, if required, the new concentrator component can also comprise an attaching mechanism.

In summary, the head of a dental care device is used as a gas/liquid sampling and pre-concentrator device for gas/liquid analysis. The chemical analysis of the molecules absorbed by the pre-concentrator is done by a second external device, not being part of the head of the dental care device itself (e.g., being in the body, in the charger of the power toothbrush, or in a brush head sanitizer). The read-out/analysis of molecules bonded to the concentrator component does not need to take place after every brushing session but rather can be after a number of brushing sessions in order to collect a higher concentration of molecules. Inside the sensor, the collected selected molecules are released from the concentrator component for analysis (as described, this may be done in many ways).

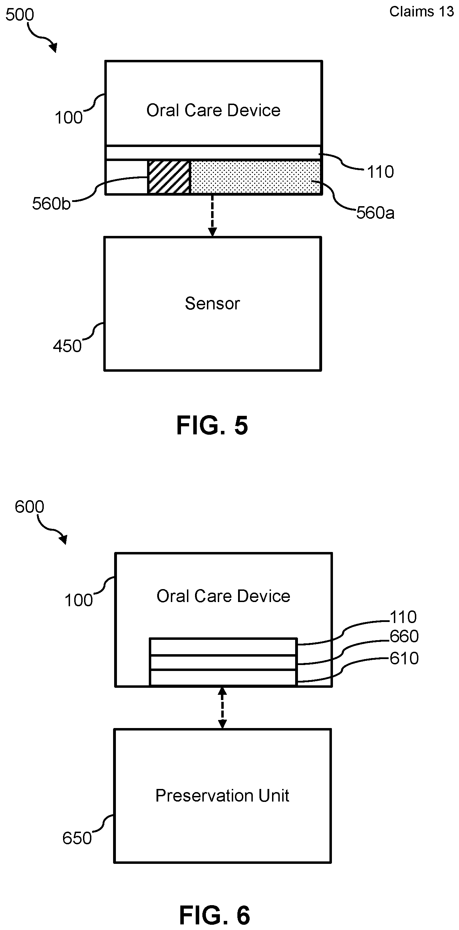

Referring now to Fig. 5, there is depicted a system 500 including a sensor 450 according to a proposed embodiment.

The system 500 comprises an oral care device 100 and a sensor 450. The oral care device 100 is an oral care device of any herein-described embodiment, and the sensor 450 is substantially the same as the sensor 450 described in Fig. 4.

In this embodiment, in addition to the concentrator component 110, the oral care device 100 comprises a protective layer, 560a and 560b, covering (at least a substantial portion of the outer surface of) the concentrator component 110 which is configured to be impermeable to the selected (i.e., target) molecules. In this embodiment, the sensor 450 is configured to remove, responsive to releasing molecules bonded with the concentrator component (i.e., afterwards), a part 560b of the protective layer, 560a and 560b, thereby exposing a corresponding part of the concentrator component 110 underneath. The part of the protective layer may be removed in any suitable way, for example, by melting it (e.g., if it is wax-based), dissolving it, mechanically removing it, moving a mechanical 'window' or punching through it (for example, in a way analogous to a medicine blister).

Referring now to Fig. 6, there is depicted a system 600 including a preservation unit 650 according to a proposed embodiment. The preservation unit 650 is a device which is configured to: receive the concentrator component 110; apply a protective layer 660 covering the concentrator component and configured to be impermeable to the selected (target) molecules; and apply a new concentrator component 610 (substantially the same as the old concentrator component 110) over the protective layer 660. In this way, a protective layer and new concentrator component are provided on another concentrator component thereby allowing the layers of concentrator components to subsequently be peeled back over time to allow a time-varying analysis of selected molecules. This is particularly beneficial when the sensor which will analyse the bonded molecules is in a separate location to the oral care device, e.g., in a remote lab, such that time-varying analysis can still be performed even if the concentrator components are only sent rarely (e.g., every few months).

The protective layer 660 may take the form of a chemical fixation layer such that the bonded molecules are captured permanently. For example, the protective layer 660 may take the form of a small droplet which forms a thin layer of cyanoacrylate over the concentrator component 110, where moisture in the air triggers polymerization. During analysis, the protective layer(s) could then be removed using acetone, for example.

As would be understood by the skilled person, if required, the preservation unit 650 can also have an attaching mechanism substantially the same as the attaching mechanism 460b of the sensor 450 of Fig. 4.

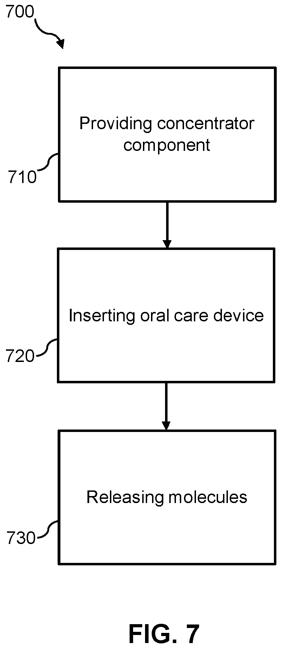

Referring now to Fig. 7, there is depicted a simplified flow diagram of a method 700 for extracting selected molecules from a mouth of a user according to a proposed embodiment.

The method 700 comprises step 710 of providing, as part of an oral care device, a concentrator component configured, whilst in or near a mouth of a user, to releasably bond with selected molecules from the mouth of the user, wherein the concentrator component is configured to be releasably attachable to a sensor configured to analyse selected molecules bonded with the concentrator component. In other words, step 710 comprises providing a concentrator component of an oral care device according to any herein-described embodiment.

Step 720 comprises inserting said oral care device into the mouth (i.e., oral cavity) of the user - this naturally leads to selected molecules present in the user's mouth bonding with the concentrator component. Step 730 finally comprises releasing the molecules bonded with the concentrator component such that, for example, they can be analysed by an appropriate sensor.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. An oral care device (100) comprising:
a concentrator component (110) configured, whilst in or near a mouth of a user, to releasably bond with selected molecules from the mouth of the user;
wherein the concentrator component is configured to be releasably attachable to a sensor configured to analyze selected molecules bonded with the concentrator component.

2. The oral care device of claim 1, wherein the concentrator component is configured, in a first state, to bond with the selected molecules, and, in a second state, to release bonded molecules, and wherein the concentrator component is configured to be in the first state whilst in or near a mouth of a user.

3. The oral care device of claim 2, wherein the concentrator component is configured to be in the second state responsive to:
exposure of the concentrator component to a predetermined surrounding temperature;
exposure of the concentrator component to a light of a predetermined intensity and/or wavelength;
exposure of the concentrator component to a predetermined surrounding air pressure;
exposure of the concentrator component to a predetermined surrounding partial pressure of the selected molecules;
exposure of the concentrator component to a solvent;
exposure of the concentrator component to a mechanical stress; and/or exposure of the concentrator component to an electric field of a predetermined strength.

4. The oral care device of any of claims 1-3, wherein the concentrator component comprises a thin film (220) configured to bond with the selected molecules, and preferably wherein the thin film comprises at least one of: a metal organic framework, MOF, material; a polymer; and/or biological anti-bodies.

5. The oral care device of any of claims 1-4, further comprising a semi-permeable membrane (230) covering the concentrator component and configured to permit passage of gas and prevent the passage of liquid.

6. The oral care device of any of claims 1-5, further comprising a temporary layer (240) covering a part of the concentrator component and configured to dissolve after a period of use of the oral care device thereby exposing the part of the concentrator component.

7. The oral care device (300) of any of claims 1-6, comprising:
a body (315);
a head (320) mounted on the body of the oral care device; and
a plurality of cleaning elements (330) mounted on the head;
wherein the concentrator component (310a, 310b) forms part of at least one of the plurality of cleaning elements, and/or wherein the concentrator component is provided on a surface of the brush head.

8. A system (400), comprising:
the oral care device (100) of any of claims 1-7; and
a sensor (450) configured to:
receive the concentrator component;
release the molecules bonded with the concentrator component; and
analyse the extracted molecules.

9. The system of claim 8, wherein the sensor and/or the concentrator component comprises an attaching mechanism (460a, 460b) configured to releasably attach and detach the sensor and concentrator component to and from one another.

10. The system of claim 8 or 9, wherein the sensor (450) is integrated with a handle of the oral care device, or the sensor is integrated with a charging device for charging the oral care device, or the sensor is integrated with a sanitizer device for cleaning the oral care device.

11. The system of any of claims 8-10, wherein the sensor is further configured, responsive to releasing molecules bonded with the concentrator component, to apply a new concentrator component to the oral care device.

12. The system of any of claims 8-11, wherein the sensor is further configured, responsive to releasing molecules bonded with the concentrator component, to remove the concentrator component or deactivate the concentrator component.

13. The system of any of claims 8-12, wherein the oral care device comprises a protective layer (560a, 560b) covering the concentrator component, and configured to be impermeable to the selected molecules; and
wherein the sensor is further configured to remove, responsive to releasing molecules bonded with the concentrator component, a part (560b) of the protective layer thereby exposing a corresponding part of the concentrator component

14. A system (600) comprising:
the oral care device (100) of any of claims 1-7; and
a preservation unit (650) configured to:
receive the concentrator component;
apply a protective layer covering the concentrator component and configured to be impermeable to the selected molecules; and
apply a new concentrator component over the protective layer.

15. A method (700) for extracting selected molecules from a mouth of a user, comprising:
providing, as part of an oral care device, a concentrator component (710) configured, whilst in or near a mouth of a user, to releasably bond with selected molecules from the mouth of the user, wherein the concentrator component is configured to be releasably attachable to a sensor configured to analyze selected molecules bonded with the concentrator component;
inserting the oral care device (720) into the mouth of the user; and
releasing the molecules (730) bonded with the concentrator component.
